(19) Europäisches Patentamt
European Patent Office
Office européen des brevets

(11) Publication number: **0 371 818 A2**

(12) # EUROPEAN PATENT APPLICATION

(21) Application number: **89312514.6**

(51) Int. Cl.$^5$: **C07K 7/00, G01N 33/00**

(22) Date of filing: **30.11.89**

(30) Priority: **01.12.88 GB 8828098**

(43) Date of publication of application:
**06.06.90 Bulletin 90/23**

(84) Designated Contracting States:
**AT BE CH DE ES FR GB GR IT LI LU NL SE**

(71) Applicant: **THE WELLCOME FOUNDATION LIMITED**
**183-193 Euston Road**
**London NW1 2BP(GB)**

(72) Inventor: **Duncan, Richard Julian Stuart**
**Wellcome Research Laboratories Langley Court**
**Beckenham Kent BR3 3BS(GB)**

(74) Representative: **Stott, Michael John et al**
**The Wellcome Research Laboratories Group Patents & Agreements Langley Court**
**Beckenham Kent BR3 3BS(GB)**

(54) **Peptides.**

(57) A synthetic peptide of the formula X-NSWGC-Y wherein

(i) X is KYLQDQARL and Y is AFRQVC, or X is AIEKYLQDQARL and Y is hydroxy, or X is K and Y is AFRQVCHTTVPWVN;

(ii) the terminal carboxy group is optionally in amide form; and

(iii) the sulphydryl group of each C is free or blocked and its use in diagnostic assays.

EP 0 371 818 A2

# PEPTIDES

The present invention relates to peptides capable of binding to antibody specific for Human Immunodeficiency Virus type 2 (HIV-2) to their preparation and to their uses.

Following the discovery of HIV-2, also known as LAV-2, there has been a need to detect the presence of this virus. A group of peptides have now been identified to which antibody specific for HIV-2 may bind, enabling the presence of such antibody and consequently the virus to be detected. These peptides can therefore be used in assays for the antibody or for HIV-2 itself. They can also be employed to raise antibody themselves.

Accordingly, the present invention provides peptides of the formula (I):

X-NSWGC-Y (I)

wherein (i) X is KYLQDQARL and Y is AFRQVC, X is AIEKYLQDQARL and Y is hydroxy, or X is K and Y is AFRQVCHTTVPWVN;

(ii) the terminal carboxy group is optionally in amide form; and (iii) the sulphydryl group of each C is free or blocked.

The amino acid residues are denoted by the one letter code (Eur. J. Biochem 138, 9-37, 1984). Preferably the terminal carboxy group is in amide form, i.e. present as -CONH2, rather than in carboxy form, i.e. present as -COOH. Also preferably, the or each side chain sulphydryl group is blocked. This prevents the formation of disulphide bonds, prevents the oxidation of cysteine residues and prevents inappropriate conjugation of the peptides to carriers.

The sulphydryl blocking group may be acetamidomethyl or may be derived from an organomercury, maleimide or disulphide blocking agent. Ellman's reagent, dithiodipyridyl, is an example of a suitable disulphide blocking agent. Preferably the blocking group is derived from N-ethylmaleimide or is acetamidomethyl and has the formula:

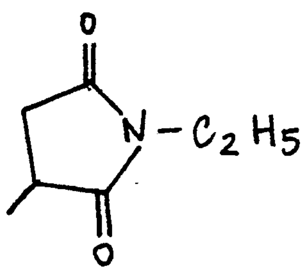 or 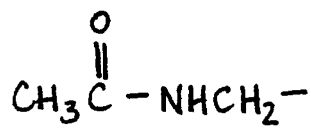

The peptides of formula (I) are synthetic peptides. They may be prepared by chemical synthesis. Solid-phase or solution methods of peptide synthesis may be employed. A peptide can be built up therefore by a process comprising:

(a) condensing single amino acids and/or preformed peptides of two or more amino acids in the order in which amino acids occur in formula (I), wherein when the amino acid is cysteine the sulphydryl group thereof is free or blocked, such as to obtain a peptide of formula (I) wherein the terminal carboxy group is free or in amide form; and

(b) if desired, blocking the free sulphydryl group of the or each cysteine residue possessing a said group in the resulting peptide of formula (I).

In solid-phase synthesis, the amino acid sequence of the desired peptide is built up sequentially from the C-terminal amino acid which is bound to an insoluble resin. When the desired peptide has been produced, it is cleaved from the resin. When solution-phase synthesis is employed, the peptide may again be built up from the C-terminal amino acid. The carboxy group of this acid remains blocked throughout by a suitable protecting group, which is removed at the end of the synthesis.

Whichever technique, solid-phase or solution-phase, is employed each amino acid added to the reaction system typically has a protected α-amino group and an activated carboxy group. An amino group may be protected by the fluoren-9-ylmethoxycarbonyl (Fmoc) or t-butoxycarbonyl (Boc) group. A carboxy group may be activated as a pentafluorophenyl or 1-oxo-2-hydroxy-dihydrobenzotriazine ester. Each condensation step may be effected in the presence of dicyclohexylcarbodiimide or 1-hydroxybenzo-triazole.

Side chain functional groups are typically protected too, for example the side chain amino group of a lysine, the side chain hydroxy group of a threonine or the side chain sulphydryl group of a cysteine. After

each step in the synthesis, the α-amino protecting group is removed.

However, any side-chain protecting groups are generally only removed at the end of the synthesis although they may be retained if desired. In the case of a protected sulphydryl group, however, the protecting group may be retained where it is required to serve a blocking function.

Where an alternative blocking group or a peptide with free sulphydryl group(s) is desired, the protecting group is removed.

The peptides may be prepared with a C-terminal carboxy or amide group as desired. In solid phase peptide synthesis, this may be determined by how the C-terminal amino acid is linked to the resin support and/or how the final peptide is cleaved from the resin. Typically the resin is a styrene and/or divinylbenzene polymer. The C-terminal amino acid may be linked to the resin via an ester linkage which can be cleaved by a strong acid such as HBr in trifluoroacetic acid or HF to give the peptide with a C-terminal carboxy group. Ammonolysis can give the corresponding amide instead.

An alternative method of obtaining a peptide amide by solid-phase synthesis is to arrange for the C-terminal amino acid of the peptide to be linked to the resin via a peptide aminobenzhydryl bond. This can be formed by coupling with dicyclohexylcarbodiimide and can be cleaved with HF, typically in the cold. For solution phase synthesis, whether a C-terminal carboxy or amide group is present may depend upon how the carboxy group of the C-terminal amino acid is blocked and, at the end of the synthesis, unblocked.

A peptide with a C-terminal carboxy group may be converted into one with a C-terminal amide group and vice versa.

The peptides may also be prepared by recombinant DNA methodologies. Thus, a DNA sequence encoding the peptide is provided. An expression vector is prepared which incorporates the DNA sequence and which is capable of expressing the peptide when provided in a suitable host.

The DNA sequence is located between translation start and stop signals in the vector. Appropriate transcriptional control elements are also provided, in particular a promoter for the DNA sequence and a transcriptional termination site.

The DNA sequence is provided in the correct frame such as to enable expression of the peptide to occur in a host compatible with the vector.

Any appropriate host-vector system may be employed. The vector may be a plasmid. In that event, a bacterial or yeast host may be used. Alternatively, the vector may be a viral vector. This may be used to transfect cells of a mammalian cell line in order to cause peptide expression.

The peptides may be provided with blocked sulphydryl groups in one of two ways. First, cysteines with blocked sulphydryl groups may be used in step (a) when building up the peptide from precursor amino acids. Second, a peptide of formula (I) may be obtained with free sulphydryl group(s) which are then blocked. A sulphydryl blocking agent is reacted with either free cysteine or the peptide of formula (I) depending on the route.

Any appropriate sulphydryl blocking agent may be used. Examples include acetamidomethanol and organomercury, maleimide and disulphide blocking agents. Ellman's reagent, dithiopyridyl, is an example of a suitable disulphide blocking agent. Preferred however is N-ethylmaleimide or acetamidomethanol.

When a peptide of formula (I) is obtained with free sulphydryl group(s) which it is then desired to block, the peptide is first reduced. A powerful reducing agent such as dithionite, 2-mercaptoethanol, dithiothreitol or dithioerythritol is typically used. Dithiothreitol is preferred. Where only a small excess of reducing agent is used, it is not necessary to separate the reduced peptide before adding the sulphydryl blocking agent.

In a typical synthesis, a solution of peptide (100-500 µM) in a buffer of about pH 7 is reduced with an approximately two-fold molar excess of a reducing agent such as dithiothreitol (i.e. 200 - 1000 µM) for at least 20 minutes, typically for about 30 mins. After this time the blocking agent such as N-ethylmaleimide is added to give an approximately two-fold molar excess over all sulphydryl present. The blocking reaction is allowed to occur for at least 1 hour. The modified peptide may be then gel-filtered into any suitable buffer solution. Alternatively, the modified peptide may be further reacted with conjugation reagents such as S-acetylthioglycolic acid N-hydroxysuccinimide ester (SATA) or 4-(N-maleimido-methyl)-cyclohexane-1-carboxylate (SMCC) before gel filtration. A peptide of formula (I) can be used in assays for antibody specific for HIV-2. A test sample of any appropriate physiological fluid may be used in the assay, for example urine, plasma, blood, serum, semen, tears, saliva or cerebrospinal fluid. The assay method comprises contacting a test sample with the peptide and determining whether any antibody binds to the peptide. For this purpose, a test kit may be provided comprising a peptide of formula (I) and means for determining whether any antibody against HIV-2 which there may be in a test sample binds to the peptide.

A variety of assay formats may be employed. The peptide can be used to capture selectively antibody against HIV-2 from solution, to label selectively such antibody already captured, or to both capture and label. In addition the peptide may be used in a variety of homogeneous assay formats in which the

antibodies which react with the peptide are detected in solution with no separation of phases. The peptide can also be used for HIV-2 antigen detection.

The types of assay in which the peptide is used to capture antibodies from solution involve immobilization of the peptide onto a solid surface. This surface should be capable of being washed in some way. The sort of surfaces which may be used are polymers of various types (moulded into microtitre wells; beads; dipsticks of various types; aspiration tips; electrodes; and optical devices), particles (for example latex; stabilized blood, bacterial or fungal cells; spores; gold or other metallic sols; and proteinaceous colloids; with the usual size of the particle being from 0.1 to 5 microns), membranes (for example nitrocellulose; paper; cellulose acetate; and high porosity/high surface area membranes of an organic or inorganic material).

The attachment of the peptide to the surfaces can be by passive adsorption from a solution of optimum composition which may include surfactants, solvents, salts, chaotropes; or by active chemical bonding. Active bonding may be through a variety of reactive or activatible functional groups which may be attached to the surface (for example condensing agents; active esters, halides, anhydrides; amino, hydroxyl, or carboxyl groups; sulphydryl groups; carbonyl groups; diazo groups; unsaturated groups).

Alternatively the active bonding may be through a protein (itself attached to the surface passively or through active bonding) or through a carrier protein such as albumin or casein, to which the peptide may be chemically bonded by any of a variety of methods and which may confer advantages because of isoelectric point, charge, hydrophilicity or other physico-chemical property. The peptide may also be attached to the surface (usually but not necessarily a membrane) following electrophoretic separation of a reaction mixture e.g. an immune precipitation.

After contacting (reacting) the surface bearing the peptide with a test sample and removing the excess of the sample where necessary by any of a variety of means (washing, centrifugation, filtration, magnetism, capilliary action), the captured antibody is detected by any means which will give a detectable signal. For example, this may be achieved by use of a labelled molecule or particle as defined above which will react with the captured antibody (for example protein A or protein G and the like; anti-species or anti-immunoglobulin-sub-type; rheumatoid factor; antibody to the peptide, used in a competitive or blocking fashion; or any molecule containing the epitope making up the peptide including the peptide itself and other proteins and peptides derived directly or indirectly from HIV-2).

The detectable signal may be optical or radio-active or physico-chemical, provided by directly labelling the molecule referred to with for example a dye, radiolabel, electroactive species, magnetically resonant species or fluorophore; or indirectly by labelling the molecule or particle with an enzyme itself capable of giving rise to a measurable change of any sort. Alternatively the detectable signal may be due to, for example, agglutination, diffraction effect or birefringent effect occurring if any of the surfaces referred to are particles.

Those types of assay in which the peptide is used to label an already captured antibody require some form of labelling of the peptide which will allow it to be detected. The labelling can be direct, by chemically or passively attaching for example a radio-, magnetic resonant-, particle or enzyme label to the peptide; or indirect by attaching any form of label to a molecule which will itself react with the peptide, e.g. antibody to the peptide, with subsequent reaction of the labelled molecule with the peptide.

The chemistry of bonding a label to the peptide can be directly through a moiety already present in the peptide, such as an amino group or through an inserted group such as a maleimide. Capture of the antibody may be on any of the surfaces already mentioned, by any reagent, including passive or activated adsorption, which will result in specific antibody or immune complexes being bound. In particular capture of the antibody could be by anti-species or anti-immunoglobulin-sub-type, by rheumatoid factor, proteins A, G and the like, or by any molecule containing the epitope making up the peptide as described above.

For those assays in which the peptide is used to provide a measure of HIV-2 antigen in a sample, the peptide may be labelled in any of the ways described above, and used in either a competitive binding fashion so that its binding by any specific molecule on any of the surfaces exemplified above is blocked by antigen in the sample, or in a non-competitive fashion when antigen in the sample is bound specifically or non-specifically to any of the surfaces above, in turn binds a specific bi- or poly-valent molecule (e.g. an antibody) and the remaining valencies of the molecule are used to capture the labelled peptide.

In general in homogeneous assays the peptide and an antibody are labelled, so that, when the antibody reacts with the peptide in free solution, the two labels interact, for example to allow non-radiative transfer of energy captured by one label to the other label, with appropriate detection of the excited second label or quenched first label (e.g. by fluorimetry, magnetic resonance or enzyme measurement). Addition of either antigen or antibody in a sample results in restriction of the interaction of the labelled pair, and so to a different level of signal in the detector.

A suitable assay format for detecting HIV-2 antibody is the direct sandwich enzyme immunoassay (EIA) format. A peptide of formula (I) is coated onto microtitre wells. A test sample and a peptide of formula (I) to which an enzyme is coupled (conjugated peptide) are added simultaneously.

Any specific antibody binds both to the peptide coating the well and to the conjugated peptide. Typically the same peptide of formula (I) is used on both sides of the sandwich. After washing, bound enzyme is detected using a specific substrate involving a colour change. A test kit for use in such an EIA comprises:

(1) a peptide of formula (I) labelled with an enzyme;

(2) a substrate for the enzyme;

(3) means providing a surface on which a peptide of formula (1) is immobilised; and

(4) optionally, washing solutions and/or buffers.

The peptides of formula (I) can be used in a combined assay for the detection of both HIV-1 and HIV-2 specific antibodies. Such an assay comprises contacting a test sample with a peptide of formula (I) and with a polypeptide which presents an epitope to which HIV-1 antibody binds and determining whether any antibody binds to the peptide of formula (I) and/or the polypeptide presenting an epitope to which HIV-1 antibody binds. Any of the assay formats defined above may be adopted.

A suitable test kit for use in a combined assay for detecting HIV-1 and HIV-2 specific antibodies comprises a peptide of formula (I), a polypeptide which presents an epitope to which HIV-1 antibody binds, and means for determining whether any antibody against HIV-1 or HIV-2 which there may be in a test sample binds to the said polypeptide or the peptide of formula (I) respectively.

The EIA format described above is suitable for use in a combined assay. A polypeptide presenting an epitope to which HIV-1 antibody binds is coated onto microtitre wells.

A said polypeptide, for example the same polypeptide, labelled with an enzyme is added simultaneously with the test sample and conjugated peptide of formula (I). The enzyme label on the polypeptide may be the same or different from that on the peptide of formula (I). As well as components (1) to (4) above, therefore, a test kit for use in such an EIA comprises:

(5) a polypeptide which presents an epitope to which HIV-1 antibody binds and which is labelled with an enzyme;

(6) a substrate for the enzyme if the enzyme is different from that labelling the peptide of formula (I); and

(7) means providing a surface on which a polypeptide which presents an epitope to which HIV-1 antibody binds is immobilised.

Where the peptide of formula (I) and the polypeptide to which HIV-1 antibody binds are immobilised on the same surface, the means referred to in (3) and (7) are the same.

The polypeptide to which HIV-1 antibody binds may be a polypeptide prepared by chemical synthesis. Alternatively, it may be a recombinant polypeptide.

Where recombinant polypeptides are provided on either side of the sandwich, they may be ones expressed in organisms of a different genus. One recombinant polypeptide may be expressed in a procaryotic organism whilst the other may be expressed in a eucaryotic organism. Examples of suitable hosts are B.subtilis, E.coli, Streptomyces, insect cells, yeasts and mammalian cells. HIV-1 provokes in particular two types of antibody. These are anti-p24 against the gag protein and anti-gp41 against the env protein. We have now prepared a specific fusion construct to which both anti-p24 and anti-gp41 bind. This protein may therefore be used as the polypeptide presenting an epitope to which HIV-1 antibody binds. The protein has the sequence:

10 20

MetAsnSerProAspThrGlyHisSerSerGlnValSerGlnAsnTyrProIleValGln

p18> p24>

30 40

AsnIleGlnGlyGlnMetValHisGlnAlaIleSerProArgThrLeuAsnAlaTrpVal

50 60

LysValValGluGluLysAlaPheSerProGluValIleProMetPheSerAlaLeuSer

70 80

GluGlyAlaThrProGlnAspLeuAsnThrMetLeuAsnThrValGlyGlyHisGlnAla

90 100

AlaMetGlnMetLeuLysGluThrIleAsnGluGluAlaAlaGluTrpAspArgValHis

110 120

ProValHisAlaGlyProIleAlaProGlyGlnMetArgGluProArgGlySerAspIle

130 140

AlaGlyThrThrSerThrLeuGlnGluGlnIleGlyTrpMetThrAsnAsnProProIle

150 160

ProValGlyGluIleTyrLysArgTrpIleIleLeuGlyLeuAsnLysIleValArgMet

170 180

TyrSerProThrSerIleLeuAspIleArgGlnGlyProLysGluProPheArgAspTyr

190 200

ValAspArgPheTyrLysThrLeuArgAlaGluGlnAlaSerGlnGluValLysAsnTrp

210 220

MetThrGluThrLeuLeuValGlnAsnAlaAsnProAspCysLysThrIleLeuLysAla

230 240

LeuGlyProAlaAlaThrLeuGluGluMetMetThrAlaCysGlnGlyValGlyGlyPro

250 260

AsnSerProArgGlnLeuLeuSerGlyIleValGlnGlnGlnAsnAsnLeuLeuArgAla

gp41>

270 280

IleGluAlaGlnGlnHisLeuLeuGlnLeuThrValTrpGlyIleLysGlnLeuGlnAla

290 300

ArgIleLeuAlaValGluArgTyrLeuLysAspGlnGlnLeuLeuGlyIleTrpGlyCys

310 320

SerGlyLysLeuIleCysThrThrAlaValProTrpAsnAlaSerTrpSerAsnLysSer

330 340

LeuGluGlnIleTrpAsnAsnMetThrTrpMetGluTrpAspArgGluIleAsnAsnTyr

350 360

ThrSerLeuIleHisSerLeuIleGluGluSerGlnAsnGlnGlnGluLysAsnGluGln

370

GluLeuLeuGluLeuAspLysTrpAlaSerLeuTrpAsnTrpPheAsnGlyAspPro.

This protein is designated DM626. Its preparation is described in our European Application No. 88308170.5. The sequence of the protein may be modified by one or more amino acid substitutions, insertions and/or deletions and/or by an extension at either or both ends provided that a protein having such a modified sequence is capable of binding to both anti-p24 and anti-gp41 and there is a degree of homology of at least 75% between the modified and the unmodified sequences.

The unmodified sequence is basically a fusion of parts of the p24 and gp41 proteins of the CBL-1 isolate of HIV-1 (WO 86/04423). These parts correspond to amino acids 121 to 356 and 542 to 674 respectively, following a similar numbering system to that of Meusing et al Nature, 313, 450-458 (1985). The start of these parts is shown above at amino acids 17 and 244 respectively. Amino acids 5 to 16 above are derived from the p18 protein. Amino acids 1 to 4, 241 to 243 and 377 to 379 above are derived from the expression vector from which the fusion construct was obtained and from DNA manipulations.

The sequence may be modified by one or more amino acid substitutions, insertions and/or deletions. These may occur anywhere in the sequence but especially in the parts of the sequence which are not derived from the p24 and gp41 proteins. In the case of substitutions, one or more of the amino acids of the unmodified sequence may be substituted by one or more other amino acid which preserves the physicochemical character of the sequence, i.e. in terms of charge density, hydrophilicity/hydrophobicity, size and configuration. For example, Ser may be replaced by Thr and vice versa, Glu may be replaced by Asp and vice versa and Gln may be replaced by Asn and vice versa. The Ser residue at amino acid 10 may be replaced by Asn.

The sequence may also be extended on one or both ends. This may be no more than the provision of an additional carboxy-terminal Cys residue. However, the sequence may be extended by up to 50 amino acid residues at either or both ends. Up to 40 amino acids, for example up to 20 amino acids, may therefore be added to the amino-terminus and/or carboxy-terminus of the unmodified sequence. The amino-terminal amino acid, however, will normally be Met due to the translational start codon of the nucleic acid sequence from which the protein is expressed.

This is unless the protein has been expressed fused at its amino-terminus to a carrier protein and the fusion protein has been cleaved to release the protein of the invention.

The sequence may be modified by introducing corresponding changes into the DNA sequence encoding the unmodified protein. This may be achieved by any appropriate technique, including restriction of the sequence with an endonuclease, insertion of linkers, use of an exonuclease and/or a polymerase and site-directed mutagenesis techniques. Whether the modified DNA sequence encodes a modified protein to which both anti-p24 and anti-gp41 are capable of binding can be readily determined. The modified sequence is cloned into an appropriate plasmid, a host cell is transformed with the plasmid and the protein that is expressed is tested for its ability to bind anti-p24 and anti-gp41. Also, there must be a degree of homology of at least 75%, for example of 85% or more or of 90% or more, between the amino acid sequences of the modified and unmodified proteins.

Antibody specific to a peptide of formula (I) can be raised using a said peptide. The antibody may be polyclonal or monoclonal. The antibody can be used in quality control testing of batches of peptide;

purification of recombinant protein, peptide or viral lysate; epitope mapping; when labelled, as a conjugate in a competitive type assay for antibody detection; and in antigen detection assays.

Polyclonal antibody can be raised by injecting a peptide of formula (I) coupled to a carrier into a mammal or other animal such as a mouse, rat, sheep or rabbit and recovering the antibody against the peptide thus produced. The peptide-carrier conjugate is generally administered as an injectable formulation comprising also a physiologically acceptable diluent. Adjuvants such as Freund's complete adjuvant (FCA) or Freund's incomplete adjuvant (FIA) may be included in the formulation. The animals are immunised over a suitable period of time. They are bled at appropriate intervals for assay for anti-peptide activity. When an appropriate level of activity is reached, the animals are bled. Antibody is extracted and purified e.g. by salt precipitation and affinity chromatography using immobilised synthetic peptide.

A hybridoma which produces monoclonal antibody can be prepared by fusing immortalising cells with cells which produce antibody against a peptide of formula (I). Typically, an animal host such as a mouse is immunised with respect to the peptide. The spleen is removed from the immunised host. Spleen cells are fused with cells of an immortal cell line such as a myeloma cell line e.g. of a mouse. In this way, a hybridoma secreting peptide-specific monoclonal antibody is produced. The antibody may be purified as above.

The following Examples illustrate the invention.

Example 1: Preparation of the peptide KYLQDQARLNSWGCAFRQVC with a carboxy-terminal amide group

The peptide was synthesised using an adaption of the Merrifield method (Merrifield, JACS **85**, 2149-2154, 1963) described by Houghten (Houghten, Proc. Natl. Acad. Sci. USA **82**, 5131-5135, 1985). The peptide was synthesised on a p-methylbenzhydrylamine divinylbenzene resin. The $\alpha$-amino protecting group on each amino acid was t-butoxycarbonyl (Boc). Each coupling cycle was as follows: 1.Wash resin with dichloromethane - 10 minutes 2.Wash with 5% diisopropylethylamine in dichloromethane - 2 minutes x 3 3.Dichloromethane wash - 1 minute x 2 4.Couple t-butoxycarbonyl amino acid in dichloromethane, 0.3M diisopropylcarbodiimide - 60 minutes 5.As 3 6.Deprotect with 50% trifluoroacetic acid in dichloromethane -20 minutes 7.Dichloromethane wash - 1 minute x 6 8.Return to 2.

When the coupling cycles were completed the peptide was cleaved off the resin using hydrogen fluoride for 1 hour with an anisole scavenger 10%. The peptide was thus obtained with a carboxy-terminal amide group. It was then ether washed, dried, dissolved in 15% acetic acid and lyophilized.

Example 2: Preparation of the peptides AIEKYLQDQARLNSWGC and KNSWGCAFRQVCHTTVPWVN each with a carboxy-terminal amide group

Each peptide was prepared as described in Example 1. Each peptide therefore had a carboxy-terminal amide group.

Example 3: Blocking of sulphydryl groups

The sulphydryl groups of each of the peptides synthesised in Examples 1 and 2 were blocked as follows. A solution of peptide (100-500 µM) in 25 mM HEPES buffer, pH 7.0, was reduced by reaction with a two-fold molar excess of dithiothreitol (i.e. 200-1000 µM) for 30 minutes. In the case of the peptide AIEKYLQDQARLNSWGC, however, a one-fold molar excess of dithiothreitol (100-500 µM) was used as the peptide has only one cysteine residue.

N-Ethylmaleimide was added to give a two-fold molar excess over all sulphydryl groups present. The blocking reaction was allowed to occur for 1 hour. The modified peptide was then gel-filtered into a buffer solution.

Example 4: Direct sandwich EIA using the peptide KYLQDQARLNSWGCAFRQVC with blocked sulphydryl groups

The peptide was coated onto microtitre wells passively. Samples of sera were then added to the prepared wells together with conjugated peptide. The enzyme of the peptide conjugate was alkaline

phosphatase. After an incubation period of about 1 hour, the wells were washed and substrate for the enzyme was added. This was nicotinamide adenine dinucleotide phosphate (NADP) with a cyclic amplification system. Anti-HIV-2 in the test samples was ascertained by comparison with a standard taken through the procedure. The results are shown in Table 1.

TABLE 1

| | Samples known to be HIV-2 +ve | Samples known to be HIV-1 +ve | Samples known to be HIV -ve |
|---|---|---|---|
| +ve in assay | 115 | 0 | 0 |
| -ve in assay | 2 | 15 | 170 |

Example 5 : Enzyme immunoassay for the detection of antibodies to Human Immunodeficiency Virus Type 1 + 2 (HIV-1 and HIV-2) using the HIV-2 peptide KYLQDQARLNSWGCAFRQVC and the HIV-1 fusion construct of the sequence referred to on pages 14-16 herein

The peptides were coated onto microtitre wells. Samples of sera were then added to the prepared wells together with conjugated peptides. The conjugates were a mixture of the same antigens which have been labelled with alkaline phosphatase. After an incubation period of about 1 hour, the wells were washed and the substrate for the enzyme was added. This was nicotinamide adenine dinucleotide phosphate (NADP) with a cyclic amplification system which results in the formation of a coloured product. After incubation the enzyme reactions were terminated and the colour was read spectrophotometrically. The amount of conjugate and hence colour, in the wells was directly related to the concentration of antibody to HIV in the Sample. The results are shown in Tables 2, 3 and 4.

TABLE 2

| Detection of Antibody to HIV-1 and HIV-2 in Serum Samples (Centres A,C,D and E) and Plasma Samples (Centre B) from European Blood Donors | | | | |
|---|---|---|---|---|
| Centre | No. of Samples Tested | Non-reactive | Initially Reactive | Repeatedly Reactive |
| A | 2064 | 2055 | 9 | 3 |
| B | 1842 | 1836 | 6 | 1 |
| C | 1897 | 1893 | 4 | 2 |
| D | 1757 | 1756 | 1 | 0 |
| E | 1557 | 1553 | 4 | 1 |
| TOTALS | 9117 | 9093 | 14(0.26%) | 7 (0.08%) |

**TABLE 3**

**European Clinical Samples**

Reactivity of Sera from Patents with AIDS, AIDS Associated Conditions, High Risk Groups and Other Diseases

| Clinical Group | No of Samples | Antibody Positive by HIV-1 and 2 | Confirmed HIV-1 Antibody Positive[a] |
|---|---|---|---|
| AIDS | 117 | 117 | 117 |
| ARC | 107 | 107 | 107 |
| High Risk[b] | 304 | 219 | 219 |
| Miscellaneous[c] | 15 | 13 | 13 |
| Diseases unrelated to AIDS[d] | 140 | 1 | 1 |

[a] Confirmation was by Western blot and/or at least two alternative immunoassays
[b] Patients in established risk groups
[c] Patients with AIDS associated conditions (e.g. Persistent Generalised Lymphadenopathy
[d] Includes patients with acute viral diseases, autoimmune disease and neoplasi[a]

## WEST AFRICAN SAMPLES

The reactivity of Wellcozyme HIV-1 and HIV-2 antibody was assessed by testing 386 West African Samples. The HIV-1 and HIV-2 test detected all samples classified as positive by immunoassays for HIV-2

antibody.

TABLE 4

| REACTIVITY OF SERA FROM WEST AFRICAN PATIENTS | | |
|---|---|---|
| No of Samples | Antibody Positive By Assay 1 + 2 | Antibody Positive By An HIV-2 Immunoassay |
| 386 | 261[a] | 259[b] |

[a] Two samples gave discordent results. One was negative in alternative tests for anti-HIV-1 and anti-HIV-2, showed only p24 in HIV-1 Western Blot but no HIV-2 Western Blot result was available. The ot er sample was negative in anti-HIV-test, equivocal in an anti-HIV-2 test and indeterminate by both HIV-1 and HIV-2 Western Blot.

[b] All samples were positive in an anti-HIV-2 immunoassay. HIV-2 Western Blots have been performed on these samples, 26 were clearly positive and 3 gave indeterminate results.

## Claims

1. A peptide of the formula (1):
X-NSWGC-Y (1)
wherein (i) X is KYLQDQARL and Y is AFRQVC, X is AIEKYLQDQARL and Y is hydroxy, or X is K and Y is AFRQVCHTTVPWVN;
(ii) the terminal carboxy group is optionally in amide form; and
(iii) the sulphydryl group of each C is free or blocked.

2. A peptide according to claim 1 wherein the terminal carboxy group is in amide form.

3. A peptide according to claim 1 or claim 2 wherein the sulphydryl group of each C is blocked using a blocking agent.

4. A peptide according to claim 3 wherein the blocking group is acetamidomethyl, dithiodipyridyl, or is derived from Ellman's reagent, or an organomercury or maleimide compound.

5. A peptide according to claim 3 wherein the blocking agent is derived from N-ethylmaleimide.

6. A peptide according to claim 1 having the amino acid sequence:
KYLQDQARLNSWGCAFRQVC

7. A peptide according to claim 1 having the amino acid sequence:
KYLQDQARLNSWGCAFRQVC
the terminal carboxy group being in amide form and the sulphydryl group of each C being blocked.

8. A method of preparing a peptide according to any of claims 1 to 7 comprising:-
(a) condensing single amino acids and/or preformed peptides of two or more amino acids in the required order, wherein, when the amino acid is cysteine, the sulphydryl group thereof is free or blocked; and
(b) if desired, converting the terminal carboxy group into amide form and, if desired, blocking the free sulphydryl group of the or each cysteine residue.

9. A method for the preparation of a peptide according to any of claims 1 to 7 which method comprises:
(i) transforming an host cell with a vector which incorporates a gene encoding a peptide according to any of claims 1 to 7 and which is capable, in the host cell, of expressing the peptide;
(ii) culturing the transformed host cell so that the peptide is expressed; and
(iii) recovering the peptide.

10. A method for determining the presence of HIV-2 antibodies in a body fluid comprising:
(a) contacting a solid phase to which is immobilised a peptide according to any of claims 1 to 7 with a test sample;
(b) means for determining whether the test sample contained any said antibody.

11. A method for determining the presence of HIV-1 or HIV-2 antibodies in a body fluid comprising:

(a) contacting a solid phase, to which are immobilised a peptide according to any of claims 1 to 7 and a polypeptide which presents an epitope to which HIV-1 antibody binds, with a test sample;

(b) means for determining whether the test sample contained any said antibodies.

12. A method according to either of claims 10 and 11 wherein the means are by use of a labelled molecule or particle.

13. A method according to claim 12 wherein the labelled molecule or particle is alkaline phosphatase, protein A, protein G, anti-species or anti-immunoglobulin sub-type, rheumatoid factor, antibody to the peptide, or any molecule containing the epitope making up the peptide.

14. A method according to claim 13 wherein the labelled molecules or particles for the detection of HIV-1 and HIV-2 are the same or different.

15. A test kit suitable for use in determining the presence of HIV-2 antibodies, which kit comprises:

(a) a peptide according to any of claims 1 to 7 labelled with an enzyme

(b) a substrate for the enzyme

(c) means providing a surface on which a peptide according to any of claims 1 to 7 is immobilised; and

(d) optionally, washing solutions and/or buffers.

16. A test kit suitable for use in a combined assay for HIV-1 and HIV-2, which kit comprises:

(a) a peptide according to any of claims 1 to 7 labelled with an enzyme;

(b) a polypeptide which presents an epitope to which HIV-1 antibody binds and which is labelled with an enzyme;

(c) substrates for both the said enzymes;

(d) means providing surfaces on which the peptide according to any of claims 1 to 7 is immobilised, and the polypeptide which presents an epitope to which HIV-1 antibody binds; and

(e) optionally, washing solutions and/or buffers.

17. A test kit according to claim 16 wherein the said HIV-1 peptide is a gag sequence of HIV-1 comprising amino acids 121 to 356 and an env sequence is an env sequence of HIV-1 comprising amino acids 542-674.